# EUROPEAN PATENT APPLICATION

(11) **EP 3 447 397 A1**
(43) Date of publication of application: **27.02.2019**
(21) Application number: 18187201.1
(22) Date of filing: 03.08.2018
(51) Int. Cl.: F24F 3/16, B01D 53/22, F04B 43/04, F04B 53/16, F24F 11/77

(54) **AIR CLEANING APPARATUS**

(30) Priority: 25.08.2017 TW 10628915
(71) Applicant: Microjet Technology Co., Ltd, Hsinchu (TW)
(72) Inventor: Mou, Hao-Jan, Hsinchu (TW); Huang, Chi-Feng, Hsinchu (TW); Han, Yung-Lung, Hsinchu (TW); Chen, Hsuan-Kai, Hsinchu (TW)
(74) Representative: Uexküll & Stolberg

(57) **Abstract**

An air cleaning apparatus (100) includes a housing (101), an actuating and sensing module (1) and an air cleaning unit (2). The housing (101) includes an inlet through hole (1011) and an outlet through hole (1012). The actuating and sensing module (1) and the air cleaning unit (2) are disposed inside the housing (101). The actuating and sensing module (1) includes an actuator (13) and a sensor (12). The actuator (13) drives external air to flow into an interior of the housing (101) via the inlet through hole (1011) and discharge the air via the outlet through hole (1012), so as to form an internal air flow. The sensor (12) detects at least one detecting target in the internal air flow to generate an air detection result. The air cleaning unit (2) includes a filter element made of a graphene material and disposed adjacent to the actuator (13), so that the internal air flow is cleaned by the filter element.

## Description

### FIELD OF THE DISCLOSURE

The present disclosure relates to an air cleaning apparatus, and more particularly to an air cleaning apparatus including a filter element made of a graphene material.

### BACKGROUND OF THE DISCLOSURE

Nowadays, the air pollution problems are becoming increasingly serious in our country and its neighboring regions. In particular, the concentration of fine suspended particles (PM 2.5) is often too high, and the public gradually starts to pay attention to the cleaning and protection of various airborne pollutants in their daily lives, so as to maintain their health and prevent from the respiratory diseases.

Currently, most of the conventional air cleaning apparatus adsorbs the pollutants in the air by means of the electrostatic principle. However, while the electrostatic filter screen meets the moisture, the static electricity of the electrostatic filter screen may weaken or disappear. It causes the blocking effect of the pollutant to be invalid. At the same time, the electrostatic filter has a certain volume, which is not conducive to the design and manufacture of a portable air cleaning apparatus.

Graphene has a high specific surface area and excellent chemical stability. As a kind of material with ultra-small pore size, it can adsorb pollutants in a purely physical way when filtering the air, so that the air cleaning efficiency is greatly improved, and it is not affected by the interference of water vapor and has long-term stability. In addition, graphene is the thinnest and lightest nanomaterial in the world. Therefore, using graphene as a filter material for the air cleaning apparatuses is beneficial to the compressed volume of the air cleaning apparatus and creates a new generation of light, thin, short and small air cleaning apparatuses for the consumers to improve the air quality around the environment anywhere and at any time.

In summary, in order to solve the problems of poor performance of the conventional air cleaning apparatuses, being subject to the external environmental factors such as the moisture, and the inability to compress the volume, there is a need of providing an air cleaning apparatus including a filter element made of a graphene material to solve the drawbacks in prior arts.

### SUMMARY OF THE DISCLOSURE

The air cleaning apparatus of the present disclosure can solve the following problems existing in the prior art: (1) the conventional air cleaning apparatuses have a poor cleaning performance for the small pollutants such as PM2.5 and are subject to the external factors such as moisture; (2) the conventional air cleaning apparatuses are too bulky to be carried around by the users to improve the air quality of the surrounding environment anytime and anywhere.

In accordance with an aspect of the present disclosure, there is provided an air cleaning apparatus including a housing, an actuator and an air cleaning unit. The housing includes an inlet through hole and an outlet through hole in communication with the external environment. The actuator and the air cleaning unit are disposed inside the housing. The actuator drives air to flow from the external environment into the interior of the housing via the inlet through hole, and discharge the air via the outlet through hole, so as to form an internal air flow inside the housing. The air cleaning unit includes a filter element made of a graphene material and is disposed adjacent to the actuator so that the air is cleaned by the filter element.

Since the air cleaning unit of the present disclosure filters the air by utilizing a filter element made of a graphene material, it can reduce the influence of the external factors such as moisture, and can significantly increase the air cleaning efficiency especially for the small-sized pollutants. At the same time, based on the physical properties of the graphene material, the air cleaning unit can be made into a miniature size, thereby compressing the structure of the air cleaning apparatus of the present disclosure. Therefore, it has the advantages of being lightweight, portable and suitable to be carried by the user.

In accordance with another aspect of the present disclosure, the air cleaning apparatus further includes an actuating and sensing module. The actuating and sensing module includes the actuator, a sensor, a microprocessor and a transmission module. The actuator can be a fluid actuator, and the fluid actuator is a micro-electro-mechanical system (MEMS) pump or a piezoelectric actuating pump. The sensor disposed adjacent to the actuator for detecting at least one detecting target contained in the air and correspondingly generating an air detection value. The microprocessor is electrically connected to the actuator, the sensor, the air cleaning unit and the transmission module. The microprocessor receives the air detection value from the sensor, processes and calculates the air detection value to generate an air detection result, and controls the air cleaning unit according the air detection result, so that the air cleaning unit is turned on, turned off, or performs an action of adjusting the intensity of its cleaning operation. The transmission module includes a positioning module and the positioning module generates position data. The transmission module transmits the air detection value and the position data to a cloud device through a communication transmission path. Thus, the cloud device can control an external air cleaning apparatus according to the air detection value, so that the external air cleaning apparatus is turned on, turned off, or performs an action of adjusting the intensity of its cleaning operation. At the same time, with a plurality of air cleaning apparatuses of the present disclosure, the transmission modules of the air cleaning apparatuses are utilized to transmit the air detection values to the cloud device and form an air-quality monitoring database through the data analysis and integration operations of the cloud device.

Since the actuator of the air cleaning apparatus of the present disclosure has a miniaturized volume, it can cooperate with the filter element made of the graphene material to miniaturize the air cleaning apparatus of the present disclosure and make the air cleaning apparatus lightweight and suitable for the user to carry around. In addition, since the actuator can continuously inhale a quantitative amount of the external air into the actuating and sensing module, a stable and consistent internal air flow is formed and detected by the sensor, so that the stability and accuracy of air detection are greatly improved. Moreover, by applying the above-mentioned innovative actuating and sensing module to the air cleaning apparatus, it is possible to monitor the air quality of the surrounding environment and automatically perform the air purification on the surrounding environment at the same time. Thus, the ambient air quality of the users is ensured to maintain anywhere and at any time.

The above contents of the present disclosure will become more readily apparent to those ordinarily skilled in the art after reviewing the following detailed description and accompanying drawings, in which:

### BRIEF DESCRIPTION OF THE DRAWINGS

FIG. 1 is a schematic structural view illustrating an air cleaning apparatus according to an embodiment of the present disclosure;
FIG. 2 is a functional block diagram illustrating an actuating and sensing module of an embodiment of the present disclosure;
FIG. 3A is a schematic exploded view illustrating an actuator of the actuating and sensing module according to an embodiment of the present disclosure;
FIG. 3B is another schematic explode view illustrating the actuator of the actuating and sensing module according the embodiment of the present disclosure at a different viewing angle;
FIG. 4 is a schematic cross-sectional view illustrating a piezoelectric actuator of the actuator of FIGS. 3A and 3B;
FIG. 5 is a schematic cross-sectional view of the actuator according to the embodiment of the present disclosure; and
FIGS. 6A to 6E are cross-sectional views illustrating processing actions of the actuator of the actuating and sensing module according to an embodiment of the present disclosure.

### DETAILED DESCRIPTION OF THE PREFERRED EMBODIMENT

The present disclosure will now be described more specifically with reference to the following embodiments. It is to be noted that the following descriptions of preferred embodiments of this disclosure are presented herein for purpose of illustration and description only. It is not intended to be exhaustive or to be limited to the precise form disclosed.

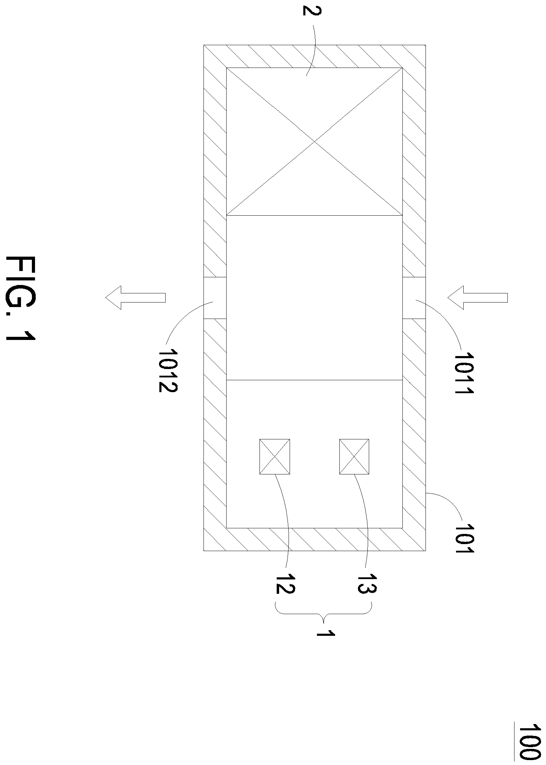

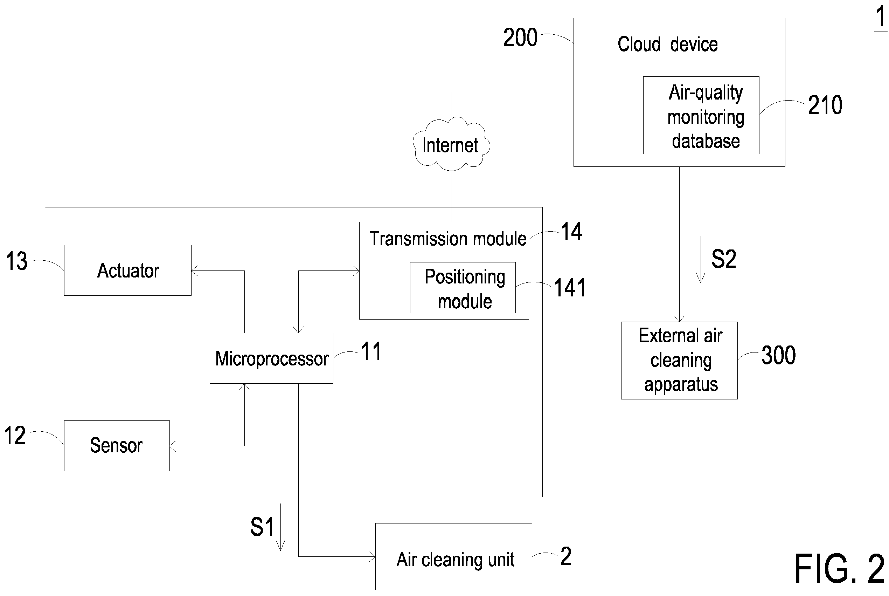

Please refer to FIGS. 1 and 2. The present disclosure provides an air cleaning apparatus 100 including at least one housing 101, at least one inlet through hole 1011, at least one outlet though hole 1012, at least one air, at least one air cleaning unit 2 and at least one filter element. The number of the housing 101, the inlet through hole 1011, the outlet through hole 1012, the air, the air cleaning unit 2 and the filter element is exemplified by one for each in the following embodiments but not limited thereto. It is noted that each of the housing 101, the inlet through hole 1011, the outlet through hole 1012, the air, the air cleaning unit 2 and the filter element can also be provided in plural numbers.

Please refer to FIG. 1. FIG. 1 is a schematic structural view illustrating an air cleaning apparatus according to an embodiment of the present disclosure. The air cleaning apparatus 100 may include a housing 101, an actuating and sensing module 1 and an air cleaning unit 2. In some other embodiments, one or more actuators may also be utilized to substitute the actuating and sensing module 1. The housing 101 has an inlet through hole 1011 and an outlet through hole 1012 which are in communication with an external environment. The inlet through hole 1011 and the outlet through hole 1012 may be disposed on two opposite sides of the housing 101 and substantially aligned with each other, but not limited thereto. The actuating and sensing module 1 and the air cleaning unit 2 are disposed inside the housing 101, and the air cleaning unit 2 is disposed at a location adjacent to the actuating and sensing module 1. In the present embodiment, the air cleaning unit 2 is disposed on one lateral side of the actuating and sensing module 1 inside the housing 101. In some other embodiments, the air cleaning unit 2 may be disposed to seal the outlet through hole 1012 or disposed inside the actuating and sensing module 1. The disposing position of the air cleaning unit 2 is not limited thereto.

The air cleaning unit 2 includes a filter element (not shown) made of a graphene material. In some embodiments, the air cleaning unit 2 further includes some other filter elements (not shown) made of another material as auxiliary. The material of said some other filter elements may be an activated carbon, a non-woven fabric, an electrostatic air filter, or a high-efficiency particulate arrestance (HEPA). Moreover, the air cleaning unit 2 includes at least one of a negative ion generator, a sterilizing light generator and a photocatalyst.

Please refer to FIG. 2. FIG. 2 is a functional block diagram illustrating an actuating and sensing module of an embodiment of the present disclosure. The actuating and sensing module 1 includes a microprocessor 11, a sensor 12, an actuator 13 and a transmission module 14. The microprocessor 11 is electrically connected with the sensor 12, the actuator 13 and the transmission module 14. The microprocessor is further electrically connected with the air cleaning unit 2 to transmit a control signal S1 thereto, so that the air cleaning unit 2 is turned on, turned off, or perform an action of adjusting intensity of cleaning operation.

The actuator 13 is a driving device capable of driving a controlled system in response to a control signal, so that the actuator 13 can drive air in the external environment to flow into the interior of the housing 101 via the inlet through hole 1011, and inhale the air into the actuating and sensing module 1. The actuator 13 includes but is not limited to an electric actuator, a magnetic actuator, a thermal actuator, a piezoelectric actuator, and a fluid actuator. For example, the electric actuator is a DC motor, an AC motor or a step motor. For example, the magnetic actuator is a magnetic coil motor, the thermal actuator is a heat pump, the piezoelectric actuator is a piezoelectric pump, and the fluid actuator is a gas pump or a liquid pump. The present disclosure is not limited thereto. The detailed structure of the actuator 13 is not redundantly described herein but will be described as follows.

The sensor 12 is disposed adjacent to the actuator 13 to detect at least one detecting target in the air inhaled by the actuator 13, and generate a corresponding air detection value. The sensor 12 may include a temperature sensor, a volatile organic compound sensor (for example, a sensor for sensing the formaldehyde and the ammonia), a particulate sensor (for example, a PM 2.5 particle sensor), a carbon monoxide sensor, a carbon dioxide sensor, an oxygen sensor, an ozone sensor, other gas sensors, a humidity sensor, a moisture sensor, a measuring sensor used for measuring the compounds and/or biological substances in water, other liquids or air (for example, a water quality sensor), other liquid sensors, a light sensor used for measuring the environment, a graphene sensor, or a group formed by any combination of the sensors described above, but is not limited thereto.

Therefore, the detecting target of the sensor 12 may be an inorganic gas or a volatile organic gas, or at least one of carbon monoxide, carbon dioxide, sulfur dioxide, nitrogen dioxide, a suspended particle, a fine suspended particle, oxygen, ozone, and any combination thereof. After the detection is over, the sensor 12 generates the corresponding air detection value accordingly. The volatile organic compound may be one of alkenes, alcohols, ketones, benzene rings, halo-alkanes, and nitrogen-containing organic compounds. In some embodiments, the sensor 12 may detect a biomarker, or detect a virus, a bacterium or a microorganism in a direct or indirect manner, but is not limited thereto.

The transmission module 14 can be a wired transmission module and a wireless transmission module and includes a positioning module 141 capable of being applied in a GPS satellite positioning system. The transmission module 14 is utilized to transmit and receive the information from an external apparatus by means of a wired communication module or a wireless communication module. The wired communication module may have an RS485 communication port, an RS232 communication port, a Modbus communication port or a KNX communication port for the wired communication path. The wireless communication module may perform the wireless communication through a Zigbee communication technology, a Z-wave communication technology, an RF communication technology, a Bluetooth communication technology, a Wifi communication technology or an EnOcean communication technology.

Please refer to FIGS. 1 and 2. After the air cleaning apparatus 100 of the present disclosure is turned on, the actuator 13 of the actuating and sensing module 1 is activated to drive the air in the external environment to flow into an interior of the housing 101 via the inlet through hole 1011, and the air is inhaled into the actuating and sensing module 1. After flowing through the sensor 12 of the actuating and sensing module 1, the air is discharged out of the housing 101 via the outlet through hole 1012. The actuator 13 drives the air to flow in and out continuously so that an internal air flow is formed inside the housing 101. The sensor 12 keeps detecting the at least one detecting target contained in the internal air flow and accordingly generates at least one air detection value. After the microprocessor 11 receives the air detection value from the sensor 12, the microprocessor 11 compares the air detection value with a preset standard value, and calculates to generate an air detection result. Then, according to the air detection result, the microprocessor 11 transmits the control signal S1 to the air cleaning unit 2 to control the air cleaning unit 2 to be turned on or turned off. Moreover, in some other embodiments, by comparing the air detection value with the preset standard value, the microprocessor 11 further generates a data comparison value. According to the data comparison value, the microprocessor 11 transmits the control signal S1 to the air cleaning unit 2 to control the air cleaning unit 2 to perform an action of adjusting intensity of cleaning operation.

For example, the sensor 12 detects a concentration of the fine suspended particles contained in the internal air flow, and generates the corresponding air detection value. The microprocessor 11 compares the air detection value with the preset standard value. If the microprocessor 11 determines that the air detection value is higher than the preset standard value, the microprocessor 11 correspondingly generates the air detection result which represents a poor air quality. At this time, the microprocessor 11 controls the air cleaning unit 2 to be turned on, so as to filter and clean the air around the air cleaning apparatus 100. Therefore, the air quality of the area around the user is improved. Meanwhile, the sensor 12 still keeps detecting the concentration of the fine suspended particles in the internal air flow. In other words, an instant air detection value is continuously transmitted to the microprocessor 11 to be compared and calculated for determination. Once the microprocessor 11 determines that the air detection value is lower than the preset standard value, the microprocessor 11 correspondingly generates the air detection result which represents a good air quality. Also, the microprocessor 11 controls the air cleaning unit 2 to be turned off. Moreover, the data comparison value is further generated after the microprocessor 11 has compared the air detection value with the preset standard value. If the microprocessor 11 determines the data comparison value is higher than a predetermined value, the microprocessor 11 accordingly controls the air cleaning unit 2 to perform the action of enhancing the intensity of its air cleaning operation. With the above actions, the air cleaning device 100 of the present disclosure can be automatically turned on or turned off, or automatically adjust the intensity of the air cleaning operation, based on the environmental condition, without the user's manual operation.

In some embodiments, in addition to the above actions, the microprocessor 11 further transmits the air detection result to the transmission module 14. After the transmission module 14 receives the air detection result, the positioning module 141 of the transmission module 14 generates position data. The air detection value and the position data are transmitted together to a cloud device 200 through a communication transmission path. The cloud device 200 can be a computer or any similar device constructed to contain CPU, RAM and other components, with a data analysis management function. In this embodiment, the cloud device 200 is serving as a server, which is connected to the transmission module 14 through the internet, and can transmit and receive the information in a wired or wireless communication transmission manner. In this way, the cloud device 200 can obtain the air detection results and the corresponding position data collected by a plurality of the air cleaning apparatuses 100, and integrate them to form an air-quality monitoring database 210. In some other embodiments, the cloud device 200 also can transmit a cloud control signal S2 according to the received air detection result to control at least one external air cleaning apparatus 300, by which the external air cleaning apparatus 300 is controlled to be turned on, turned off or to perform the action of adjusting the intensity of cleaning operation. The external air cleaning apparatus 300 may be any device constructed to improve the air quality such as an air cleaner, a dehumidifier, an exhaust vent fan, an automatic door, an automatic window, an automatic cleaning robot, an air conditioner, etc. The present disclosure is not limited thereto.

The detailed structure of the actuator 13 is described as follows. In the embodiment, the actuator 13 of the actuating the sensing module 1 may be a driving structure of a piezoelectric actuating pump or a driving structure of a micro-electro-mechanical system (MEMS) pump. The following is an illustration of the processing actions of a piezoelectric actuating pump taken as an example of the actuator 13.

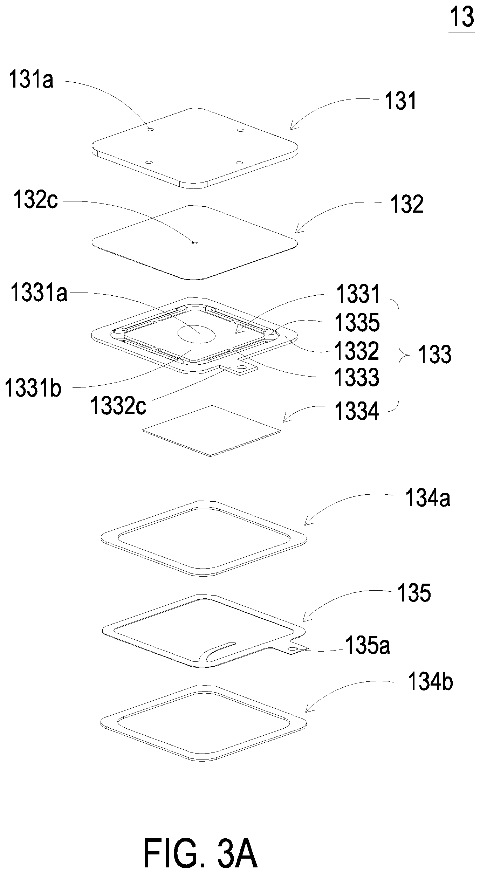

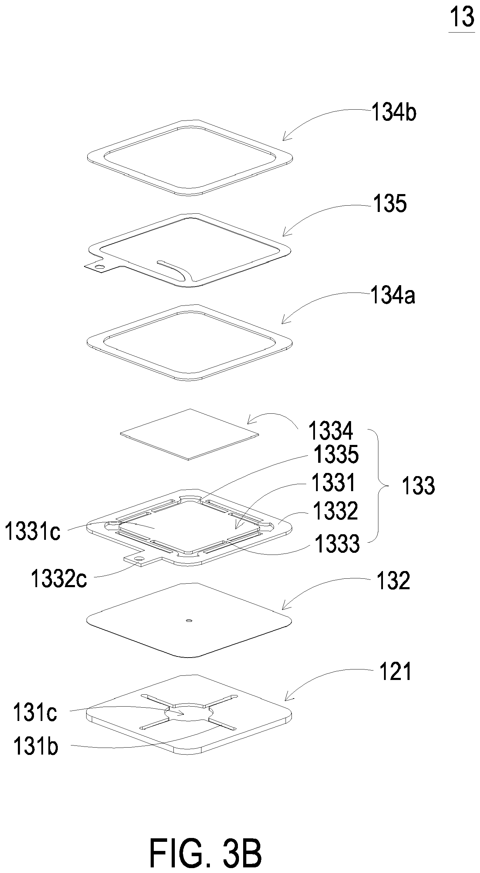

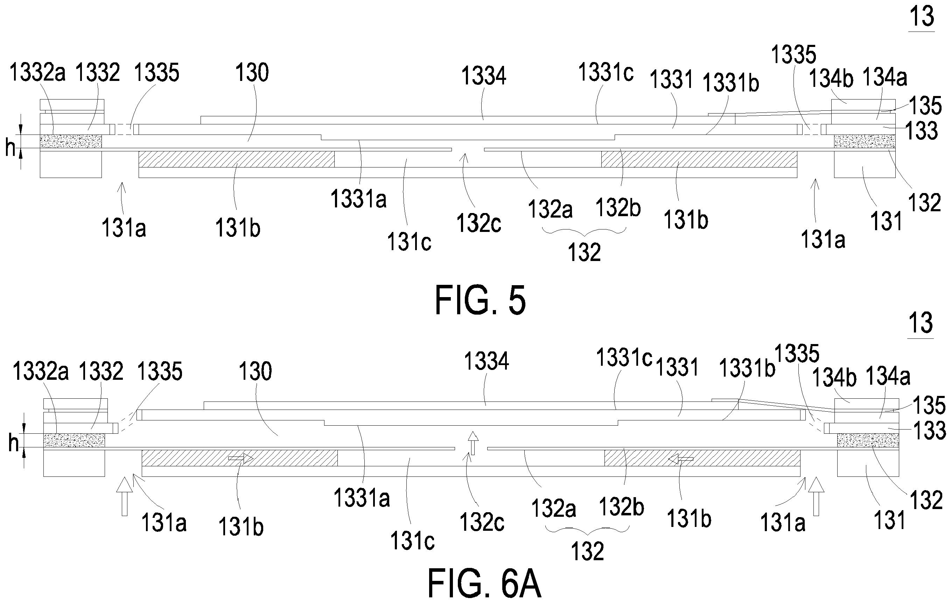

Please refer to FIGS. 3A and 3B. FIG. 3A is a schematic exploded view illustrating an actuator of the actuating and sensing module according to an embodiment of the present disclosure; and FIG. 3B is another schematic explode view illustrating the actuator of the actuating and sensing module according the embodiment of the present disclosure at a different viewing angle. The actuator 13 includes an air inlet plate 131, a resonance plate 132, a piezoelectric actuator 133, a first insulation plate 134a, a conducting plate 135 and a second insulation plate 134b. The piezoelectric actuator 133 is aligned with the resonance plate 132. The air inlet plate 131, the resonance plate 132, the piezoelectric actuator 133, the first insulation plate 134a, the conducting plate 135 and the second insulation plate 134b are stacked on each other sequentially. After the above components are combined together, the cross-sectional view of the resulting structure of the actuator 13 is shown in FIG. 5.

In the embodiment, the air inlet plate 131 has at least one inlet 131a. Preferably but not exclusively, the air inlet plate 131 has four inlets 131a. The inlets 131a run through the air inlet plate 131. In response to the action of the atmospheric pressure, the air can be introduced into the actuator 13 through the at least one inlet 131a. Moreover, at least one convergence channel 131b is formed on a surface of the air inlet plate 131, and is disposed spatially corresponding to the at least one inlet 131a on another surface of the air inlet plate 131. Moreover, a central cavity 131c is located at the intersection of the convergence channels 131b. The central cavity 131c is in communication with the at least one convergence channel 131b, such that the air entered by the at least one inlet 131a would be introduced into the at least one convergence channel 131b and is guided to the central cavity 131c. Consequently, the air can be transferred by the actuator 13. In this embodiment, the at least one inlet 131a, the at least one convergence channel 131b and the central cavity 131c of the air inlet plate 131 are integrally formed in one piece from a single structure. The central cavity 131c is a convergence chamber for temporarily storing the air. In some embodiments, the air inlet plate 131 may be, for example, made of stainless steel. Moreover, the depth of the convergence chamber defined by the central cavity 131c is equal to the depth of the at least one convergence channel 131b. The resonance plate 132 is made of a flexible material. The resonance plate 132 has a central aperture 132c spatially corresponding to the central cavity 131c of the air inlet plate 131, so as to allow the air to flow therethrough. In other embodiments, the resonance plate 132 may be, for example, made of copper, but not limited thereto.

The piezoelectric actuator 133 includes a suspension plate 1331, an outer frame 1332, at least one bracket 1333 and a piezoelectric plate 1334. The piezoelectric plate 1334 is attached on a first surface 1331c of the suspension plate 1331. In response to an applied voltage, the piezoelectric plate 1334 would be subjected to a deformation. When the piezoelectric plate 1334 is subjected to the deformation, it facilitates a bending vibration of the suspension plate 1331. In this embodiment, the at least one bracket 1333 is connected between the suspension plate 1331 and the outer frame 1332, while the two ends of the bracket 1333 are connected with the outer frame 1332 and the suspension plate 1331 respectively that the bracket 1333 can elastically support the suspension plate 1331. At least one vacant space 1335 is formed among the bracket 1333, the suspension plate 1331 and the outer frame 1332. The at least one vacant space 1335 is in communication with an air flow channel for allowing the air to go through. It has to be emphasized that the type of the suspension plate 1331 and the outer frame 1332 and the type and the number of the at least one bracket 1333 may be varied according to the practical requirements. The outer frame 1332 is arranged around the suspension plate 1331. Moreover, a conducting pin 1332c is protruded outwardly from the outer frame 1332 so as to provide the function of electrical connection, but the present disclosure is not limited thereto.

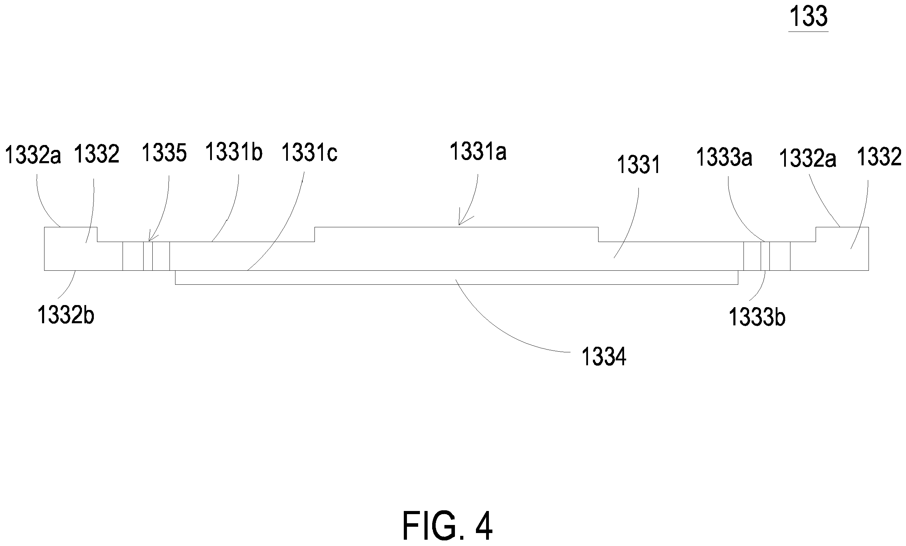

FIG. 4 is a schematic cross-sectional view illustrating a piezoelectric actuator of the actuator of FIGS. 3A and 3B. As shown in FIG. 4, the suspension plate 1331 has a bulge 1331a that makes the suspension plate 1331 a stepped structure. The bulge 1331a is formed on a second surface 1331b of the suspension plate 1331. The bulge 1331a can be for example but not limited to a circular convex structure. A top surface of the bulge 1331a of the suspension plate 1331 is coplanar with a second surface 1332a of the outer frame 1332, while the second surface 1331b of the suspension plate 1331 is coplanar with a second surface 1333a of the bracket 1333. Moreover, there is a drop of specified amount from the bulge 1331a of the suspension plate 1331 and the second surface 1332a of the outer frame 1332 to the second surface 1331b of the suspension plate 1331 and the second surface 1333a of the bracket 1333. A first surface 1331c of the suspension plate 1331, a first surface 1332b of the outer frame 1332 and a first surface 1333b of the bracket 1333 are coplanar with each other. The piezoelectric plate 1334 is attached on the first surface 1331c of the suspension plate 1331. In some other embodiments, the suspension plate 1331 may be a square plate structure with two flat surfaces, but the type of the suspension plate 1331 may be varied according to the practical requirements. In this embodiment, the suspension plate 1331, the at least bracket 1333 and the outer frame 1332 may be integrally formed and produced from a metal plate, which can be for example but not limited to a stainless steel material. In an embodiment, the length of a side of the piezoelectric plate 1334 is smaller than the length of a side of the suspension plate 1331. In another embodiment, the length of a side of the piezoelectric plate 1334 is equal to the length of a side of the suspension plate 1331. Similarly, the piezoelectric plate 1334 is a square plate structure corresponding to the suspension plate 1331 in terms of the design.

Please refer to FIG. 3A. In this embodiment, the actuator 13 includes the first insulation plate 134a, the conducting plate 135 and the second insulation plate 134b, which are stacked on each other sequentially and located under the piezoelectric actuator 133. The profiles of the first insulation plate 134a, the conducting plate 135 and the second insulation plate 134b substantially match the profile of the outer frame 1332 of the piezoelectric actuator 133. In some embodiments, the first insulation plate 134a and the second insulation plate 134b are made of an insulating material, for example but not limited to a plastic material, so as to provide insulating efficacy. In other embodiments, the conducting plate 135 may be made of an electrically conductive material, for example but not limited to a metallic material, so as to provide electrically conducting efficacy. In this embodiment, the conducting plate 135 may have a conducting pin 135a disposed thereon so as to provide the function of electrical connection.

Please refer to FIG. 5. FIG. 5 is a schematic cross-sectional view of the actuator according to the embodiment of the present disclosure. In an embodiment, the air inlet plate 131, the resonance plate 132, the piezoelectric actuator 133, the first insulation plate 134a, the conducting plate 135 and the second insulation plate 134b of the actuator 13 are stacked on each other sequentially. Moreover, there is a gap h between the resonance plate 132 and the outer frame 1332 of the piezoelectric actuator 133. In this embodiment, the gap h between the resonance plate 132 and the outer frame 1332 of the piezoelectric actuator 133, may be filled with a filler, for example but not limited to a conductive adhesive, so that a depth from the resonance plate 132 to the bulge 1331a of the suspension plate 1331 of the piezoelectric actuator 133 can be maintained. The gap h ensures the proper distance between the resonance plate 132 and the bulge 1331a of the suspension plate 1331 of the piezoelectric actuator 133, so that the air can be transferred quickly, the contact interference is reduced and the generated noise is largely reduced. In some embodiments, alternatively, the height of the outer frame 1332 of the piezoelectric actuator 133 is increased, so that the gap is formed between the resonance plate 132 and the piezoelectric actuator 133, but the present disclosure is not limited thereto.

Please refer to FIG. 3A, FIG. 3B and FIG. 5. After the air inlet plate 131, the resonance plate 132 and the piezoelectric actuator 133 are combined together, a movable part 132a and a fixed part 132b of the resonance plate 132 are defined. A convergence chamber for converging the air is defined by the movable part 132a of the resonance plate 132 and the air inlet plate 131 collaboratively. Moreover, a first chamber 130 is formed between the resonance plate 132 and the piezoelectric actuator 133 for temporarily storing the air. Through the central aperture 132c of the resonance plate 132, the first chamber 130 is in communication with the central cavity 131c of the air inlet plate 131. The peripheral regions of the first chamber 130 are in communication with the air flow channel through the vacant space 1335 between the brackets 1333 of the piezoelectric actuator 133.

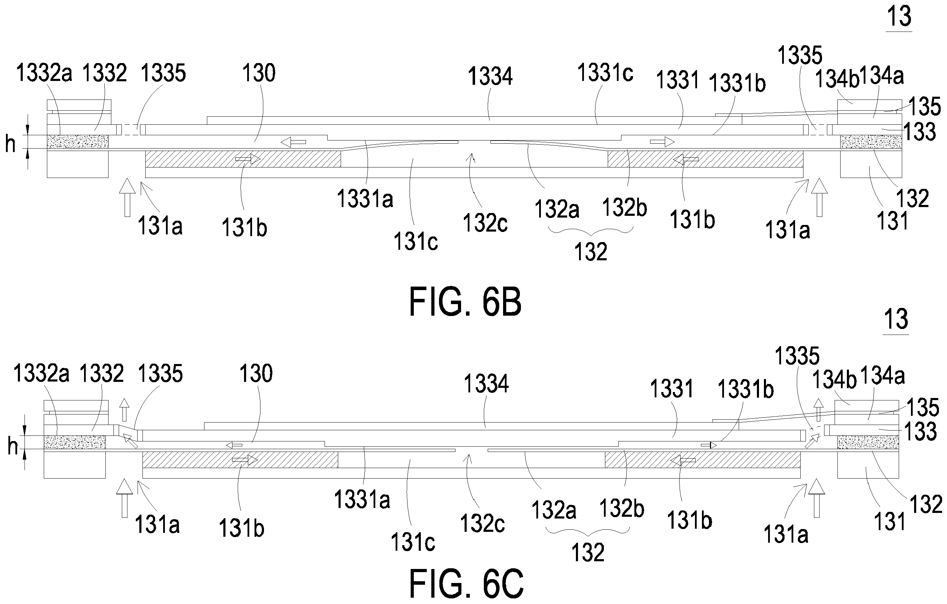

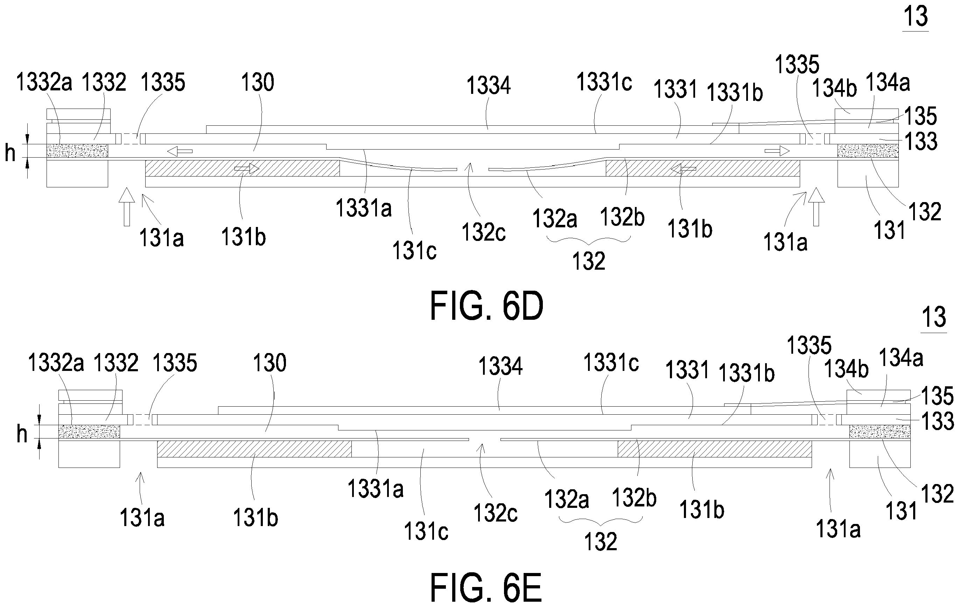

Please refer to FIGS. 3A, 3B, 5 and 6A to 6B. FIGS. 6A to 6E are cross-sectional views illustrating processing actions of the actuator of the actuating and sensing module according to an embodiment of the present disclosure. Please refer to FIG. 3A, FIG. 3B, FIG. 5 and FIGS. 6A to 6E. The actions of the actuator 13 will be described as follows. When the actuator 13 is enabled, the piezoelectric actuator 133 vibrates along a vertical direction in a reciprocating manner by using the bracket 1333 as a fulcrum. Please refer to FIG. 6A, the piezoelectric actuator 133 vibrates downwardly in response to the applied voltage. Since the resonance plate 132 is light and thin, the resonance plate 132 vibrates along the vertical direction in resonance with the piezoelectric actuator 133. More especially, a region of the resonance plate 132 spatially corresponding to the central cavity 131c of the air inlet plate 131 is also subjected to a bending deformation. The region of the resonance plate 132 corresponding to the central cavity 131c of the air inlet plate 131 is the movable part 132a of the resonance plate 132. When the piezoelectric actuator 133 vibrates downwardly, the movable part 132a of the resonance plate 132 is subjected to the bending deformation because the movable part 132a of the resonance plate 132 is pushed by the air and vibrates in response to the piezoelectric actuator 133. In response to the downward vibration of the piezoelectric actuator 133, the air is fed into the at least one inlet 131a of the air inlet plate 131. Then, the air is transferred to the central cavity 131c of the air inlet plate 131 through the at least one convergence channel 131b. Then, the air is transferred through the central aperture 132c of the resonance plate 132 spatially corresponding to the central cavity 131c, and introduced downwardly into the first chamber 130. As the piezoelectric actuator 133 is enabled, the resonance of the resonance plate 132 occurs. Consequently, the resonance plate 132 vibrates along the vertical direction in the reciprocating manner. As shown in FIG. 6B, during the vibration of the movable part 132a of the resonance plate 132 at this stage, the movable part 132a of the resonance plate 132 moves down to contact and attach on the bulge 1331a of the suspension plate 1331 of the piezoelectric actuator 133, and a distance from the fixed part 132b of the resonance plate 132 to a region of the suspension plate 1331 except the bulge 1331a remains the same. Owing to the deformation of the resonance plate 132 described above, a middle communication space of the first chamber 130 is closed, and the volume of the first chamber 130 is compressed. Under this circumstance, the pressure gradient occurs to push the air in the first chamber 130 moving toward peripheral regions of the first chamber 130 and flowing downwardly through the vacant space 1335 of the piezoelectric actuator 133. Referring to FIG. 6C, the movable part 132a of the resonance plate 132 has returned to its original position when the piezoelectric actuator 133 vibrates upwardly. Consequently, the volume of the first chamber 130 is continuously compressed to generate the pressure gradient which makes the air in the first chamber 130 continuously pushed toward peripheral regions. Meanwhile, the air is continuously fed into the at least one inlet 131a of the air inlet plate 131, and transferred to the central cavity 131c. Then, as shown in FIG. 6D, the resonance plate 132 moves upwardly, which is cause by the resonance of the upward motion of the piezoelectric actuator 133. That is, the movable part 132a of the resonance plate 132 is also vibrated upwardly. Consequently, it decreases the flow of the air from the at least one inlet 131a of the air inlet plate 131 into the central cavity 131c. At last, as shown in FIG. 6E, the movable part 132a of the resonance plate 132 has returned to its original position. As the embodiments described above, when the resonance plate 132 vibrates along the vertical direction in the reciprocating manner, the gap h between the resonance plate 132 and the piezoelectric actuator 133 is helpful to increase the maximum displacement along the vertical direction during the vibration. In other words, the configuration of the gap h between the resonance plate 132 and the piezoelectric actuator 133 can increase the amplitude of vibration of the resonance plate 132. Consequently, a pressure gradient is generated in the air flow channels of the actuator 13 to facilitate the air to flow at a high speed. Moreover, since there is an impedance difference between the feeding direction and the exiting direction, the air can be transmitted from the inlet side to the outlet side. Even if a gas pressure exists at the outlet side, the actuator 13 still has the capability of pushing the air to the air flow channel while achieving the silent efficacy. The steps of FIGS. 6A to 6E may be done repeatedly. Consequently, the ambient air is transferred by the actuator 13 from the outside to the inside.

From the above descriptions, the present disclosure provides an air cleaning apparatus, which utilizes the actuator of the actuating and sensing module to generate a flow of the air, in which the air in an external environment is inhaled via the inlet through hole and discharged via the outlet through hole, so as to form an internal air flow inside the housing. The air cleaning unit utilizes the filter element made of the graphene material to filter the internal air flow, so that the air cleansing apparatus of the present disclosure can improve the effect of air cleaning and can achieve a miniaturized volume that is convenient for the user to carry around. It meets the needs of the user to improve the ambient air quality at anytime and anywhere.

## Claims

1. An air cleaning apparatus (100), comprising:
a housing (101) having an inlet through hole (1011) and an outlet through hole (1012) for allowing air to flow from the exterior of the housing (101) to the interior of the housing (101); and
an air cleaning unit (2) disposed inside the housing (101) and having a filter element made of a graphene material to clean the air.

2. The air cleaning apparatus according to claim 1, further comprising an actuating and sensing module (1), wherein the actuating and sensing module (1) comprises:
at least one actuator (13) driving the air to flow from the exterior of the housing (101) to the interior of the housing (101) via the inlet through hole (1011) and driving the air to be discharged via the outlet through hole (1012), such that an internal air flow is generated to make the air cleaning unit (2) clean the air by the filter element; and
at least one sensor (12) disposed adjacent to the actuator (13) to measure the air and generate an air detection value.

3. The air cleaning apparatus according to claim 2, wherein the actuating and sensing module (1) further comprises a microprocessor (11) electrically connected with the actuator (13), the sensor (12) and the air cleaning unit (2), wherein the microprocessor (11) receives the air detection value from the sensor (12) and compares the air detection value with a preset standard value to calculate and generate an air detection result, and a control signal (S1) is transmitted to the air cleaning unit (2) according to the air detection result to turn on or turn off the air cleaning unit (2).

4. The air cleaning apparatus according to claim 3, wherein the microprocessor (11) compares the air detection value with the preset standard value to calculate and generate a data comparison value, and the control signal (S1) is transmitted to the air cleaning unit (2) according to the data comparison value to control the air cleaning unit (2) to perform an action of adjusting intensity of cleaning operation.

5. The air cleaning apparatus according to claim 3, wherein the actuating and sensing module (1) further comprises a transmission module (14) electrically connected with the microprocessor (11), wherein the transmission module (14) is a wired transmission module or a wireless transmission module, wherein after the microprocessor (11) transmits the air detection result to the transmission module (14), the transmission module (14) transmits the air detection result to a cloud device (200) through a communication transmission path, wherein the cloud device (200) controls at least one external air cleaning apparatus (300) according to the air detection result, so that the external air cleaning apparatus (300) is turned on, turned off or performs an action of adjusting intensity of cleaning operation.

6. The air cleaning apparatus according to claim 5, wherein the transmission module comprises a positioning module (141) generating position data, wherein the transmission module (14) transmits the air detection result and the position data to the cloud device (200) through the communication transmission path to form an air-quality monitor database (210).

7. The air cleaning apparatus according to claim 2, wherein the air detection value is a value obtained by detecting at least one selected from the group consisting of carbon monoxide, carbon dioxide, sulfur dioxide, nitrogen dioxide, suspended particle, fine suspended particle, oxygen, ozone and a combination thereof.

8. The air cleaning apparatus according to claim 2, wherein the air detection value is a value obtained by detecting a volatile organic compound, wherein the volatile organic compound comprises at least one selected from the group consisting of alkenes, alcohols, ketones, benzene rings, halo-alkanes and nitrogen-containing organic compounds.

9. The air cleaning apparatus according to claim 2, wherein the air detection value is a value obtained by detecting at least one selected from the group consisting of an inorganic gas, a biomarker, a virus, a bacterium and a microorganism.

10. The air cleaning apparatus according to claim 2, wherein the sensor (12) comprises at least one selected from the group consisting of an oxygen sensor, a carbon monoxide sensor, a carbon dioxide sensor, a temperature sensor, a liquid sensor, a moisture sensor, an ozone sensor, a fine particle sensor, a volatile organic compound sensor, a light sensor and a combination thereof.

11. The air cleaning apparatus according to claim 2, wherein the sensor (12) comprises a graphene sensor.

12. The air cleaning apparatus according to claim 1, wherein the actuator is a micro-electro-mechanical system pump.

13. The air cleaning apparatus according to claim 2, wherein the actuator (13) is a piezoelectric actuating pump and the piezoelectric actuating pump comprises:
an air inlet plate (131) having at least one inlet (131a), at least one convergence channel (131b) and a central cavity (131c) defining a convergence chamber, wherein the at least one inlet (131a) allows the air to flow in, and the convergence channel (131b) is spatially corresponding to the inlet (131a) and guides the air from the inlet (131a) toward the convergence chamber defined by the central cavity (131c);
a resonance plate (132) having a central aperture (132c) and a movable part (132a), wherein the central aperture (132c) is spatially corresponding to the convergence chamber and the movable part (132a) surrounds the central aperture (132c); and
a piezoelectric actuator (133) facing the resonance plate (132), wherein a gap (h) is formed between the resonance plate (132) and the piezoelectric actuator (133) to define a first chamber (130), so that the air from the at least one inlet (131a) of the air inlet plate (131) is converged to the central cavity (131c) along the at least one convergence channel (131b) and flows into the first chamber (130) through the central aperture (132c) of the resonance plate (132) when the piezoelectric actuator (133) is enabled , whereby the air is further transferred through a resonance between the piezoelectric actuator (133) and the movable part (132a) of the resonance plate (132).

14. The air cleaning apparatus according to claim 13, wherein the piezoelectric actuator (133) comprises:
a suspension plate (1331) being a square suspension plate and having a first surface (1331c), a second surface (1331b) and a bulge (1331a), wherein the suspension plate (1331) is permitted to undergo a bending vibration;
an outer frame (1332) arranged around the suspension plate (1331);
at least one bracket (1333) connected between the suspension plate (1331) and the outer frame (1332) for elastically supporting the suspension plate (1331); and
a piezoelectric plate (1334), wherein a length of a side of the piezoelectric plate (1334) is smaller than or equal to a length of a side of the suspension plate (1331), and the piezoelectric plate (1334) is attached on the first surface (1331c) of the suspension plate (1331), wherein when a voltage is applied to the piezoelectric plate (1334), the suspension plate (1331) is driven to undergo the bending vibration.

15. An air cleaning apparatus (100), comprising:
at least one housing (101) having at least one inlet through hole (1011) and at least one outlet through hole (1012) for allowing air to flow from the exterior of the housing (101) to the interior of the housing (101); and
at least one air cleaning unit (2) disposed inside the housing (101) and having at least one filter element made of at least one graphene material to clean the air.
